# FASCICULE DE BREVET EUROPEEN

(11) **EP 2 260 033 B1**
(45) Date de publication et mention de la délivrance du brevet: **23.11.2011**
(21) Numéro de dépôt: 08873262.3
(22) Date de dépôt: 31.12.2008
(51) Int. Cl.: C07D 471/04, A61K 31/437, A61P 19/00, A61P 35/00, A61P 25/00

(54) **Dérivés de 2-benzoyl-imidazo[1,2-a]pyridine, leur préparation et leur application en thérapeutique**
2-Benzoylimidazo[1,2-a]pyridinderivate, deren Herstellung und deren therapeutische Verwendung
2-benzoylimidazo[1,2-a]pyridine derivatives, preparation thereof and therapeutic use thereof

(30) Priorité: 02.01.2008 FR 0800007
(43) Date de publication de la demande: 15.12.2010
(73) Titulaire: SANOFI, 75013 Paris (FR)
(72) Inventeur: PEYRONEL, Jean-François, F-75013 Paris (FR)
(74) Mandataire: Le Coupanec, Pascale A.M.P.
(86) Numéro de dépôt international: PCT/FR2008/001837
(87) Numéro de publication internationale: WO 2009/112652

(56) Documents cités:
- EP-A- 1 726 585
- WO-A-2008/003854
- FR-A- 2 638 161

## Description

La présente invention se rapporte à des dérivés de 2-benzoyl-imidazo[1,2-*a*]pyridine, à leur préparation et à leur application en thérapeutique dans le traitement ou la prévention de maladies impliquant les récepteurs nucléaires Nurr-1, aussi appelés NR4A2, NOT, TINUR, RNR-1, et HZF3.

La présente invention a pour objet les composés de formule (I) : 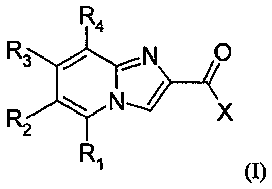 dans laquelle :
- X: représente un groupe phényle éventuellement substitué par un ou plusieurs atomes ou groupes choisis indépendamment les uns des autres parmi les atomes ou groupes suivants : halogène, (C₁-C₆)alkyle, (C₁-C₆)alcoxy, hydroxy, amino, NRaRb ; les groupes (C₁-C₆)alkyle et (C₁-C₆)alcoxy étant éventuellement substitués par un ou plusieurs atomes d'halogène ;
- R₂: représente un groupe hétérocyclique, éventuellement substitué par un ou plusieurs groupes choisis indépendamment les uns des autres parmi les atomes ou groupes suivants : hydroxy, halogène, (C₁-C₆)alcoxy, (C₁-C₆)alkyle éventuellement substitué par un ou plusieurs hydroxy, NRcRd, -CO-R₅, -CO-NR₆R₇, -CO-O-R₈, -NR₉-CO-R₁₀, cyano, un groupe oxido ;
- R₁: représente un atome d'hydrogène, un halogène, un groupe (C₁-C₆)alcoxy, un groupe (C₁-C₆)alkyle, hydroxy, amino; les groupes (C₁-C₆)alkyle et (C₁-C₆)alcoxy pouvant éventuellement être substitués par un ou plusieurs atomes ou groupes halogène, hydroxy, amino, (C₁-C₆)alcoxy;
- R₃: représente un atome d'hydrogène, un atome d'halogène, un groupe (C₁-C₆)alkyle, ou un groupe hydroxy ;
- R₄: représente un atome d'hydrogène ou un atome d'halogène ;
- R₅: représente un atome d'hydrogène ou un groupe (C₁-C₆)alkyle ;
- R₆: et R₇, identiques ou différents, représentent un atome d'hydrogène ou un groupe (C₁-C₆)alkyle ou forment avec l'atome d'azote qui les porte un cycle de 4 à 7 chaînons incluant éventuellement un autre hétéroatome choisi parmi N, O ou S ;
- R₈: représente un groupe (C₁-C₆)alkyle ;
- R₉ et R₁₀,: identiques ou différents, représentent un atome d'hydrogène ou un groupe (C₁-C₆)alkyle; Ra représente un (C₁-C₆)alkyle ; Rb, Rc et Rd représentent un hydrogène ou un (C₁-C₆)alkyle ;
à l'état de base ou de sel d'addition à un acide.

On connaît du document FR 2 638 161 les composés dérivés de benzoyl-2-imidazo[1,2-a]pyridine, utiles en tant que médicaments.

On connaît également du document EP 1 726 585 des composés imidazo [1,2-*a*]pyridinyles, utiles dans le traitement des maladies neurodégénératives, des traumatismes cérébraux, des maladies psychiatriques et de la maladie de Parkinson.

WO 2008/003856 décrit la préparation et l'utilisation de dérivés d'imidazo [1,2-*a*]pyridine-2-carboxamides.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels peuvent être préparés avec des acides pharmaceutiquement acceptables, mais les sels d'autres acides utiles, par exemple, pour la purification ou l'isolement des composés de formule (I) font également partie de l'invention.

Dans le cadre de la présente invention, on entend par:
- un atome d'halogène : un fluor, un chlore, un brome ou un iode ;
- un groupe alkyle : un groupe aliphatique saturé linéaire, ramifié ou cyclique, éventuellement substitué par un groupe alkyle saturé linéaire, ramifié ou cyclique. A titre d'exemples, on peut citer les groupes méthyle, éthyle, propyle, isopropyle, butyle, isobutyle, tertbutyle, cyclopropyle, cyclobutyl, cyclopentyle, cyclohexyle, méthylcyclopropyl etc ;
- un groupe (C₁-C₆)alcoxy: un radical -O-alkyle où le groupe alkyle est tel que précédemment défini ;
- un groupe hétérocyclique : groupe mono ou bicyclique saturé ou insaturé ou partiellement insaturé comportant de 5 à 10 atomes dont de 1 à 4 hetéroatomes choisis parmi N, O et S. A titre d'exemples de groupes hétérocycliques, on peut citer de manière non limitative : pyrrole, furane, thiophène, pyrazole, imidazole, triazole, tétrazole, oxazole, isoxazole, oxadiazole, thiazole, isothiazole, thiadiazole, pyridine, pyrimidine, pyrazine, pyridazine, triazine, furofurane, thiénothiophène, pyrrolopyrrole, pyrroloimidazole, pyrrolopyrazole, pyrrolotriazole, imidazoimidazole, imidazopyrazole, furopyrcole, furoimidazole, furopyrazole, furotriazole, pyrrolo-oxazole, imidazo-oxazole, pyrazolo-oxazole, furo-oxazole, oxazolo-oxazole, oxazoloisoxazole, pyrrolo-isoxazole, imidazo-isoxazole, pyrazolo-isoxazole, isoxazolo-isoxazole, furo-isoxazole, isoxazolo-oxadiazole, pyrrolo-oxadiazole, furo-oxadiazole, isoxazolo-oxadiazole, thiénopyrrole, thiénoimidazole, thiénopyrazole, thiénotriazole, pyrrolo-thiazole, imidazo-thiazole, pyrazolo-thiazole, triazolo, thiazole, furo-thiazole, oxazolo-thiazole, oxazoloisothiazole, pyrrolo-isothiazole, imidazo-isothiazole, pyrazolo-isothiazole, isoxazolo-isothiazole, furo-isothiazole, pyrrolo-thiadiazole, imidazo-thiadiazole, furo-thiadiazole, isoxazolo-thiadiazole, oxazolo-thiadiazole, isothiazolo-thiadiazole, indole, isoindole, benzimidazole, indazole, indolizine, benzofurane, isobenzofurane, benzothiophène, benzo[c]thiophène, pyrrolopyridine" imidazopyridine, pyrazolopyridine, triazolopyridine, tetrazolopyridine, pyrrolopyrimidine, imidazopyrimidine, pyrazolopyrimidine, pyrrolopyrazine, imidazopyrazine, pyrazolopyrazine, pyrrolopyridazine, imidazopyridazine, pyrazolopyridazine, triazolopyridazine, pyrrolotriazine, furopyridine, furopyrimidine, furopyrazine, furopyridazine, furotriazine, oxazolopyridine, oxazolopyrimidine, oxazolopyrazine, oxazolopyridazine, isoxazolopyridine, isoxazolopyrimidine, isoxazolopyrazine, isoxazolopyridazine, oxadiazolopyridine, benzoxazole, benzisoxazole, benzoxadiazole, thiénopyridine, thiénopyrimidine, thiénopyrazine, thiénopyridazine, thiénotriazine, thiazolopyridine, thiazolopyrimidine, thiazolopyrazine, thiazolopyridazine, isothiazolopyridine, isothiazolopyrimidine, isothiazolopyrazine, isothiazolopyridazine, thiadiazolopyridine, thiadiazolopyrimidine, benzothiazole, benzoisothiazole, benzothiadiazole, quinoline, isoquinoline, cinnoline, phthalazine, quinoxaline, quinazoline, naphthyridine, benzotriazine, pyridopyrimidine, pyridopyrazine, pyridopyridazine, pyridotriazine, pyrimidopyrimidine, pyrimidopyrazine, pyrimidopyridazine, pyrazinopyrazine, pyrazinopyridazine, pyrazinotriazine, pyridazinopyridazine ; ces groupes pouvant être saturés ou partiellement insaturés ; à l'exception des hétérocycles monocycliques monoazotés de 4 à 7 chaînons contenant éventuellement un autre hétéroatome choisi parmi N, S, O et lié par l'azote.

Parmi les composés de formule (I) objets de l'invention, un premier groupe de composés est constitué des composés pour lesquels :
X représente un groupe phényle ;
R₁, R₃ et R₄ sont des atomes d'hydrogène ;
R₂ représente un groupe hétérocyclique monocyclique insaturé comportant 5 ou 6 atomes dont de 1 à 2 hétéroatomes choisis parmi N ou O, ledit groupe hétérocyclique étant éventuellement susbtitué par un groupe -NR_{c}R_{d}, R_{c} et R_{d} représentant un hydrogène ou un (C₁-C₆)alkyle.

Parmi les composés de formule (I) objets de l'invention, un second groupe de composés est constitué des composés pour lesquels :
X représente un groupe phényle ;
R₁, R₃ et R₄ sont des atomes d'hydrogène ;
R₂ représente un groupe pyridine, pyrrole, pyrazole, imidazole ou furane, éventuellement substitué par un groupe NH₂,
à l'état de base ou de sel d'addition à un acide.

Parmi les composés de formule (I) objet de l'invention, un troisième groupe de composés est constitué par les composés pour lesquels R₂ représente un groupe hétérocyclique, mono ou bicyclique saturé ou insaturé ou partiellement insaturé comportant de 5 à 10 atomes dont de 1 à 4 hétéroatomes choisis parmi N, O et S, à l'exception des hétérocycles monocycliques monoazotés de 4 à 7 chaînons contenant éventuellement un autre hétéroatome choisi parmi N, S, O et lié par l'azote.

Parmi les composés de formule (I) objets de l'invention, un quatrième groupe de composés est constitué par les composés pour lesquels R₂ représente un groupe hétérocyclique, monocyclique insaturé ou partiellement saturé comportant de 5 à 7 atomes dont de 1 à 3, notamment de 1 à 2, hétéroatomes choisis parmi O N, S, en particulier N ou O.

Parmi les composés de formule (I) objets de l'invention, un cinquième groupe de composés est constitué par les composés pour lesquels R₂ représente un groupe pyrrole, furane, thiophène, pyrazole, imidazole, triazole, tétrazole, oxazole, isoxazole, oxadiazole, thiazole, isothiazole, thiadiazole, pyridine, pyrimidine, pyrazine, pyridazine, triazine et plus particulièrement un groupe pyridine, pyrrole, pyrazole, imidazole, furane.

Parmi les composés de formule (I) objets de l'invention, on peut notamment citer les composés suivants :
- [6-(6-Aminopyridin-2-yl)imidazo[1,2-*a*]pyridin-2-yl](phényl)méthanone,
- Phényl(6-pyridin-2-ylimidazo[1,2-*a*]pyridin-2-yl)méthanone et son dichlorhydrate,
- Phényl[6-(1H-pyrrol-3-yl)imidazo[1,2-*a*]pyridin-2-yl]méthanone,
- Phényl[6-(1H-pyrazol-4-yl)imidazo[1,2-*a*]pyridin-2-yl]méthanone,
- [6-(1H-imidazol-4-yl)imidazo[1,2-*a*]pyridin-2-yl](phényl)méthanone,
- [(6-Furan-2-yl)imidazo[1,2-*a*]pyridin-2-yl](phényl)méthanone,
- Phényl[(6-pyridin-3-yl)imidazo[1,2-*a*]pyridin-2-yl]méthanone,
ou un sel d'addition de ces composés à un acide pharmaceutiquement acceptable.

Conformément à l'invention, on peut préparer les composés de formule générale (I) selon le procédé décrit dans le schéma 1.

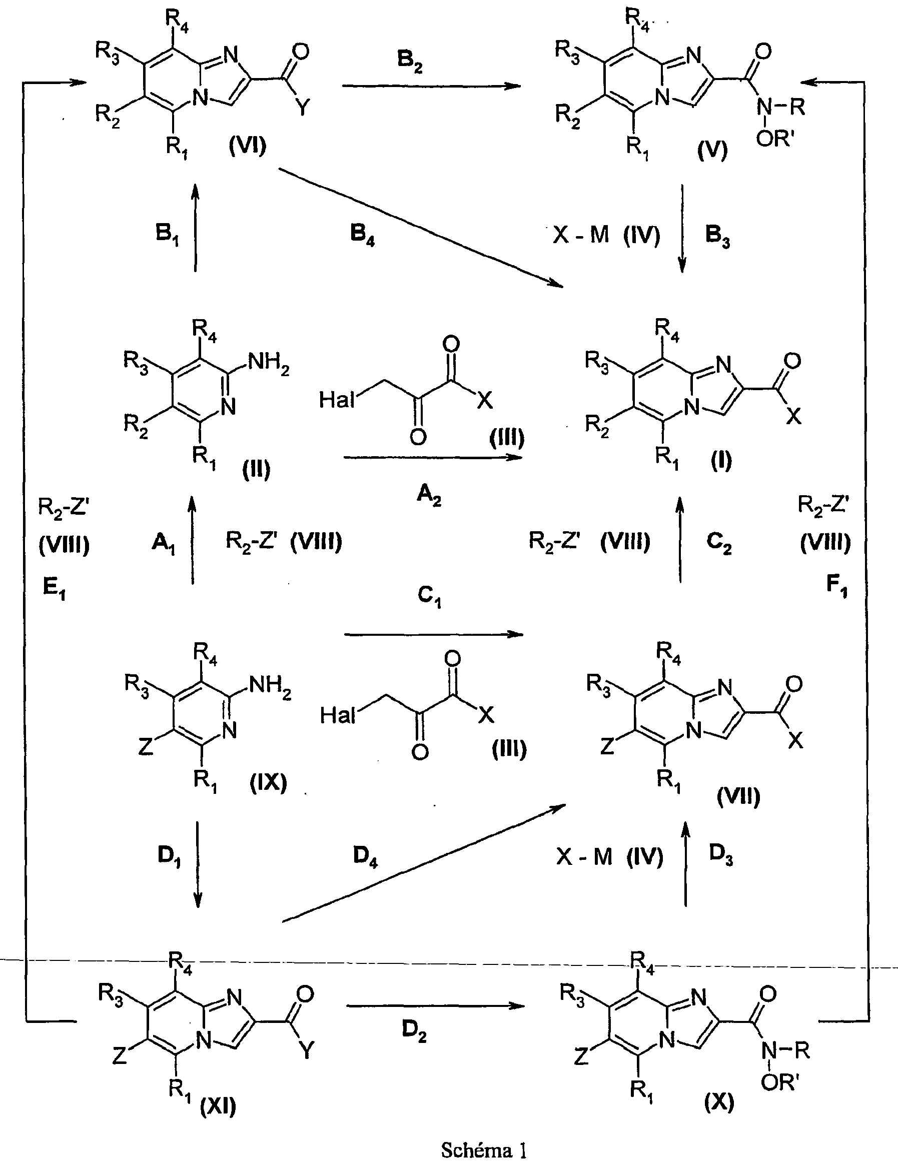

La première voie de synthèse (transformation A₂) consiste à condenser une 2-amino-pyridine de formule (II), dans laquelle R₁, R₂, R₃ et R₄ sont définis comme précédemment, sur un dérivé de 3-halogèno-1-aryl-propane-1,2-dione de formule générale (III), dans laquelle Hal représente un atome de chlore, de brome ou d'iode et X est défini comme précédemment, pour former le cycle imidazo[1,2-a]pyridine par exemple selon la méthode décrite par J-J. Bourguignon et coll. dans Aust J. Chem., 50, 719 (1997)

La seconde voie de synthèse (transformations B₃ ou B₄) consiste à faire réagir un dérivé organométallique de formule générale (IV) dans laquelle X est défini comme précédemment et M représente un atome de lithium ou un groupe Mg-Hal sur:
- un amide de Weinreb (ou N-alcoxy-N-alkyl-amide) de formule générale (V), dans laquelle R₁, R₂, R₃ et R₄ sont définis comme précédemment et sont différents de brome ou iode et R et R' - identiques ou différents - représentent un groupe alkyle, selon des méthodes connues de l'homme du métier telles que décrites par Weinreb, S. M. et al. dans Tetrahedron Letters (1981), 22(39), 3815-18 et dans Sibi, M.P. Organic Preparations and Procedures Int. 1993, 25, 15-40 (transformation B₃), ou
- sur un acide imidazo[1,2-a]pyridine-2-carboxylique de formule générale (VI), dans laquelle R₁, R₂, R₃ et R₄ sont définis comme précédemment et sont différents de brome ou iode et Y représente un hydroxy ou un de ses sels ou dérivés réactifs tels que ester, halogénure d'acide, anhydride ou amide selon des méthodes connues de l'homme du métier, telles que décrites dans J. March, Advanced Organic Chemistry (Wiley, 5th Ed. 2001) p 567 et 1213 ou dans les références citées (transformation B₄).

Alternativement, la transformation B₄ peut être réalisée en faisant réagir un dérivé réactif tel qu'un anhydride mixte (qui peut être généré *in situ*) de acide imidazo[1,2-*a*]pyridine-2-carboxylique de formule (VI), dans laquelle Y représente un hydroxy et R₁, R₂, R₃ et R₄ sont définis comme précédemment et sont différents de brome ou iode sur un dérivé organométallique de formule (IV), dans laquelle X est défini comme ci-dessus et M représente un groupe boronique en présence d'un catalyseur au palladium tel que le tétrakistriphénylphosphine palladium.

La troisième voie de synthèse (transformation C₂) consiste à réaliser le couplage catalytique d'un dérivé de formule générale (VII) dans laquelle R₁, R₃ et R₄ sont définis comme précédemment et Z représente un groupe boryle, stannyle ou silyle avec un dérivé R₂-Z' de formule (VIII), dans laquelle Z' représente un atome d'halogène tel que brome ou iode ou un groupe sulfonyloxy et R₂ est un groupe 1-alcényle, 1-alcynyle, aryle ou hétéroaryle, éventuellement substitué. Alternativement, le couplage peut être réalisé entre un dérivé de formule générale (VII), dans laquelle R₁, R₃ et R₄ sont définis comme précédemment et Z représente un atome d'halogène tel que brome ou iode, avec un dérivé R₂-Z' (VIII), dans lequel Z' représente un groupe réactif tel qu'un groupe boryle, stannyle ou silyle ou un atome d'hydrogène et R₂ est un groupe 1-alcényle, 1-alcynyle, aryle ou hétéroaryle, éventuellement substitué.

Les 2-amino-pyridines de formule (II) peuvent être préparées selon les méthodes décrites dans la littérature ou connues de l'homme du métier. En particulier, les 2-amino-pyridines de formule (II), dans laquelle R₁, R₃ et R₄ sont définis comme précédemment et R₂ est un groupe 1-alcényle, 1-alcynyle, aryle ou hétéroaryle, éventuellement substitué peuvent être préparées par la transformation A₁, c'est à dire par réaction de couplage catalytique,
- soit d'un dérivé de 2-amino-pyridine de formule (IX), dans laquelle R₁, R₃ et R₄ sont définis comme précédemment et Z représente un groupe boryle, stannyle ou silyle avec un dérivé R₂-Z' (VIII) dans lequel Z' représente un atome d'halogène tel que brome ou iode ou un groupe sulfonyloxy et R₂ est un groupe 1-alcényle, 1-alcynyle, aryle ou hétéroaryle, éventuellement substitué,
- soit d'un dérivé de 2-aminopyridine de formule (IX) dans laquelle R₁, R₃ et R₄ sont définis comme précédemment et Z représente un atome d'halogène tel que brome ou iode avec un dérivé R₂-Z' (VIII) dans lequel Z' représente un groupe réactif tel qu'un groupe boryle, stannyle ou silyle ou un atome d'hydrogène et R₂ est un groupe 1-alcényle, 1-alcynyle, aryle ou hétéroaryle, éventuellement substitué.

Les dérivés de 3-halogèno-1-aryl-propane-1,2-dione de formule (III) peuvent être préparés par halogénation des 1-aryl-propane-1,2-diones correspondantes selon les méthodes connues de l'homme de métier.

Les amides de Weinreb de formule (V) peuvent être obtenus (transformation B₂) par couplage d'un acide de formule (VI) dans laquelle Y représente un groupe hydroxy ou de l'un de ses dérivés réactifs avec une N,O-dialkylamine selon les méthodes connues de l'homme de métier. Le couplage peut être réalisé en présence d'un agent de couplage tel que CDI, EDCI, HATU ou HBTU et d'une base telle que la diisopropyléthylamine, la triéthylamine ou la pyridine, dans un solvent inerte tel que le THF, le DMF ou le dichlorométhane. Alternativement, on peut faire réagir la N,O-dialkylamine avec un ester de formule (VI), dans laquelle Y représente un groupe alcoxy, en présence d'un catalyseur tel que le triméthylaluminium (Weinreb. S. M. et al., Synth. Commun. 1982, 12, 989).

Les dérivés des acides imidazopyridine-2-carboxyliques de formule (VI), dans laquelle R₁, R₂, R₃ et R₄ sont définis comme précédemment et Y représente un groupe (C₁-C₆)alcoxy, hydroxy ou un atome d'halogène peuvent être préparés par condensation d'une 2-aminopyridine de formule (II), dans laquelle R₁, R₂, R₃ et R₄ sont définis comme précédemment sur un ester d'acide 3-halogèno 2-oxo-propionique de formule (VIII), dans laquelle Hal représente un atome de chlore, de brome ou d'iode et Y représente un groupe (C₁-C₆)alcoxy, dans les conditions décrites par J.G. Lombardino dans J. Org. Chem., 30, 2403 (1965) par exemple, suivie le cas échéant de la conversion de l'ester en acide puis en chlorure d'acide ou autre dérivé réactif (transformation B₁).

Les dérivés d'imidazo[1,2-*a*]pyridine de formule (VII), dans laquelle X, R₁, R₃ et R₄ sont définis comme précédemment et Z représente un atome d'halogène, un groupe boryle, stannyle ou silyle peuvent être préparés (transformation C₁) par condensation d'une 2-aminopyridine de formule (II), dans laquelle Z, R₁, R₃ et R₄ sont définis comme ci-dessus, sur un dérivé de 3-halogèno-1-aryl-propane-1,2-dione de formule générale (III), dans laquelle Hal représente un atome de chlore, de brome ou d'iode, dans les conditions décrites ci-dessus pour la préparation des produits de formule générale (I) par la transformation A₂.

Alternativement, les dérivés d'imidazo[1,2-*a*]pyridine de formule (VII), dans laquelle X, R₁, R₃ et R₄ sont définis comme précédemment et Z représente un atome d'halogène, un groupe boryle, stannyle ou silyle, peuvent être préparés par réaction d'un dérivé organométallique de formule générale (IV), dans laquelle X est défini comme précédemment et M représente un atome de lithium ou un groupe Mg-Hal, sur un acide imidazo[1,2-a]pyridine-2 carboxylique de formule (XI), dans laquelle R₁, R₂, R₃, R₄ et Z sont définis comme ci-dessus et sont différents de brome ou iode et Y représente un groupe hydroxy, ou d'un de ses dérivés réactifs tel que chlorure d'acide (transformation D₄), ou sur un amide de Weinreb correspondant de formule (X) (transformation D₃), en protégeant éventuellement les autres fonctions réactives dans les conditions décrites ci-dessus pour la préparation des produits de formule générale (I) par les transformations B₃ou B₄.

Les dérivés des acides imidazopyridine-2-carboxyliques de formule (X) et (XI) peuvent être préparés par condensation d'une 2-aminopyridine de formule (IX), dans laquelle Z, R₁, R₃ et R₄ sont définis comme précédemment, sur un ester d'acide 3-halogèno 2-oxo-propionique de formule (VIII), dans laquelle Hal représente un atome de chlore, de brome ou d'iode et Y représente un groupe (C₁-C₆)alcoxy, selon les méthodes décrites ci-dessus pour la préparation des dérivés de formule (V) et (VI) (transformation D₁).

Les couplages des dérivés de formule (VII), (IX) ou (X) avec les produits de formule (VIII) peuvent être effectués par toute méthode connue de l'homme de métier en particulier en opérant en présence de catalyseurs à base de cuivre ou de palladium, de ligands tels que des phosphines, selon ou par analogie avec les méthodes décrites par exemple dans les références suivantes et références citées :
- pour les réactions de type Suzuki : N. Miyaura, A. Suzuki, Chem. Rev., 95, 2457, (1995),
- pour les réactions de type Stille : V. Farina et coll., Org. React., 50.1 (1997),
- pour les réactions de type Hiyama : T. Hiyama et coll., Top. Curr. Chem., 2002, 219, 61 (2002),
- pour les réactions de type Negishi : E. Negishi et coll., Chem. Rev., 103, 1979 (2003),
- pour les réactions de type Bellina : M. Miura et coll., Chem. Lett., 200 (2007).

Il est également possible pour effectuer le couplage de former intermédiairement, mais sans les isoler, des dérivés organométalliques tels que des dérivés zinciques.

Conformément à l'invention, on peut également préparer les composés de formule générale (I), (II) et (VI) selon les procédés décrits dans le schéma 2, c'est-à-dire par la conversion d'un composé de formules générales (XII), (XIII) ou (XIV), dans lesquelles R₁, R₃, R₄ et X sont définis comme précédemment, Y représente un groupe hydroxy, alcoxy ou *N*-alcoxy-*N*-alkyl-amino et W représente un groupement précurseur permettant la construction de l'hétérocycle de formule R₂ respectivement en composés de formule générale (I), (VI) et (II) selon les méthodes connues de l'homme du métier (transformations G₁, G₂ et G₃).

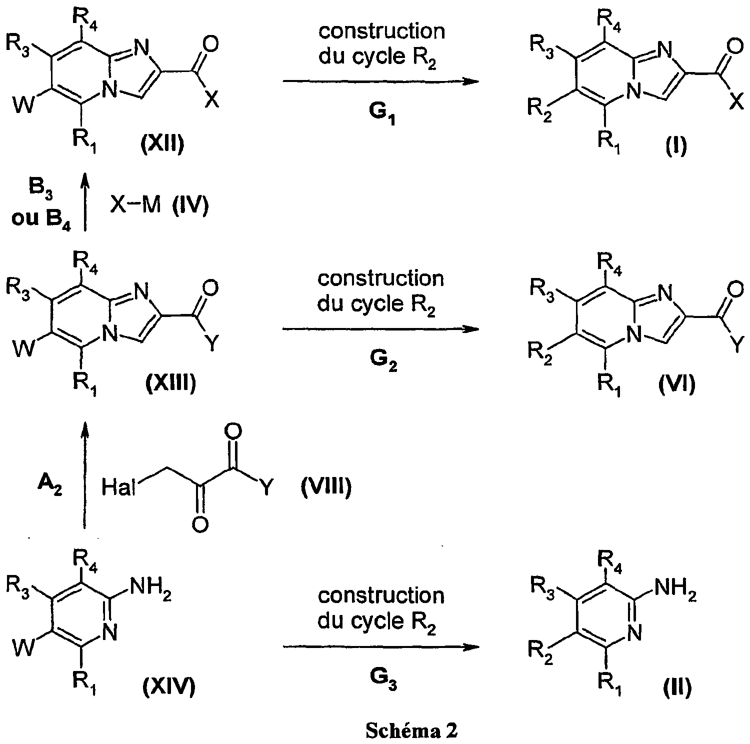

A titre d'exemple W peut représenter :
- un groupement 2-halogéno-acyle tel que bromoacétyle, ou 1-halo-2-oxo-alkyle tel que 1-bromo-2-oxo-éthyle qui peut être converti, par exemple, en groupe thiazolyle, imidazolyle, oxazolyle par traitement par des dérivés de thiourée, de thioamide, de guanidine, d'urée ou d'amide,
- un groupement alkynyle, tel qu'éthynyle qui peut être converti en groupe 1,2,3-triazol-4-yl,
- un groupement cyano qui peut être converti, par exemple, en groupe dihydroimidazolyl(2) ou 1,3,4-triazol-2-yl.

Les composés de formule générale (XII) peuvent être obtenus à partir des composés de formule (XIII), dans les conditions décrites pour la préparation des composés (I), à partir des dérivés d'acide imidazopyridine-2-carboxyliques de formules (V) ou (VI), par les transformations B₂ ou B₄.

Les dérivés d'acide imidazopyridine-2-carboxyliques de formule générale (XIII) peuvent être obtenus à partir des amino-pyridines de formule (XIV), dans les conditions décrites pour la conversion des amino-pyridines de formule (II) en composés de formules générale (I) par la transformation A₂.

Les produits de formule (I) et leurs précurseurs de formule (II), (V) ou (VI), peuvent être soumis, si désiré et si nécessaire, pour obtenir des produits do formule (I) ou être transformés en d'autres produits de formule (I), à l'une ou plusieurs des réactions de transformations suivantes, dans un ordre quelconque :
a) une réaction d'estérification ou d'amidification de fonction acide,
b) une réaction d'hydrolyse de fonction ester en fonction acide,
c) une réaction de transformation de fonction hydroxyle en fonction alcoxy,
d) une réaction d'oxydation de fonction alcool en fonction aldéhyde ou cétone,
e) une réaction d'oxydation de groupe alcènyle en fonction aldéhyde ou cétone,
f) une réaction de deshydratation de groupe hydroxyalkyle en groupe alcényle,
g) une réaction d'hydrogénation totale ou partielle de groupe alcényle ou alcynyle en groupe alcényle ou alkyle,
h) une réaction de couplage catalytique d'un dérivé halogèné et d'un dérivé organométallique tel que stannique ou boronique pour introduire un substituant alkyle, alcènyle, alcynyle, aryle ou hétéroaryle,
i) une réaction de conversion d'un dérivé halogèné pour introduire un substituant boryle, stannyle ou silyle,
j) une réaction de protection des fonctions réactives,
k) une réaction d'élimination des groupements protecteurs que peuvent porter les fonctions réactives protégées,
l) une réaction de salification par un acide minéral ou organique ou par une base pour obtenir le sel correspondant,
m) une réaction de dédoublement des formes racémiques en énantiomères, lesdits produits de formule (I) ainsi obtenus étant le cas échéant sous toutes les formes isomères possibles racémiques, énantiomères et diastéréoisomères.

Dans le schéma 1, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'Homme du métier.

Les exemples suivants décrivent la préparation de certains composés conformes à l'invention. Les numéros des exemples renvoient à ceux donnés dans les tableaux ci-après, qui illustrent les structures chimiques et les caractéristiques spectroscopiques de quelques composés selon l'invention.

### Exemple 1 : [6-(6-Aminopyridin-2-yl)imidazo[1,2-a]pyridin-2-yl](phényl)méthanone

Dans un tube à microondes on charge 300 mg de phényl[6-(4,4,5,5-tétraméthyl-1,3,2-dioxaborolan-2-yl)imidazo[1,2-*a*]pyridin-2-yl]méthanone, 123 mg de 2-amino-6-bromo-pyridine, 30 mg de tétrakis(triphénylphosphine)palladium, 2 mL de solution 2M de carbonate de sodium, 4 mL de toluène et 4 mL d'acétonitrile. Le mélange réactionnel est chauffé 20 minutes dans l'appareil à microondes réglé sur 150°C, puis refroidi, filtré sur célite, dilué par de l'acétate d'éthyle, séché sur sulfate de magnésium et concentré sous pression réduite. Le résidu est chromatographié sur une cartouche de silice en éluant par un mélange de dichlorométhane et d'acétate d'éthyle (50/50). Les fractions contenant le produit attendu sont réunies et concentrées à sec sous pression réduite. Le solide est trituré dans un mélange de dichlorométhane et d'éther isopropylique puis séché pour donner 57 mg de [6-(6-aminopyridin-2-yl)imidazo[1,2-*a*]pyridin-2-yl](phényl)méthanone sous la forme d'un solide brun.

### Exemple 2 : Chlorhydrate (2 :1) de phényl[(6-pyridin-2-yl)imidazo[l,2-a]pyridin-2-yl]méthanone

### 2.1 : Phényl[6-(pyridin-2-yl)imidazo[1,2-a]pyridin-2-yl]méthanone

Dans un tube à microondes on charge 200 mg de phényl[6-(4,4,5,5-tétraméthyl-1,3,2-dioxaborolan-2-yl)imidazo[1,2-*a*]pyridin-2-yl]méthanone, 130 mg de 2-iodo-pyridine, 26 mg de tétrakis(triphénylphosphine)palladium, 2 mL de solution 2M de carbonate de sodium, 4 mL de toluène et 4 mL d'acétonitrile. Le mélange réactionnel est chauffé 20 minutes dans l'appareil à microondes réglé sur 150°C, puis refroidi, filtré sur célite et concentré sous pression réduite. Le résidu est chromatographié sur une cartouche de silice en éluant par un mélange de dichlorométhane et d'acétate d'éthyle (75/25). Les fractions contenant le produit attendu sont réunies et concentrées à sec sous pression réduite. Le solide est trituré dans du méthanol puis filtré pour donner 95 mg de phényl(6-pyridin-2-ylimidazo[1,2-*a*]pyridin-2-yl)méthanone sous la forme d'un solide écru.

### 2.2 : Chlorhydrate (2:1) de phényl[6-(pyridin-2-yl)imidazo[1,2-a]pyridin-2-yl]méthanone

A une solution de 95 mg de phényl[6-(pyridin-2-yl)imidazo[1,2-*a*]pyridin-2-yl]méthanone dans le minimum de dioxanne on ajoute 200 µL dune solution 4M d'acide chlorhydrique dans le dioxanne. Le précipité formé est essoré, lavé à l'éther éthylique et séché pour donner 94 mg de dichlorhydrate de phényl(6-pyridin-2-ylimidazo[1,2-*a*]pyridin-2-yl)méthanone sous la forme d'un solide beige.

### Exemple 3 : Phényl[6-(1H-pyrrol-3-yl)imidazo[1,2-a]pyridin-2-yl]méthanone

### 3.1 : Phényl[6-(1-triisopropylsilyl-pyrrol-3-yl)imidazo[1,2-a]pyridin-2-yl]méthanone

Dans un tube à microondes on charge 225 mg (6-iodolimidazo[1,2-a]pyridin-2-yl)(phényl)méthanone, 225 mg d'acide 1-triisopropylsilyl-pyrrol-3-boronique, 30 mg de tétrakis-(triphénylphosphine)palladium, 2 mL de solution 2M de carbonate de sodium, 4 mL de toluène et 4 mL d'acétonitrile. Le mélange réactionnel est chauffé 20 minutes dans l'appareil à microondes réglé sur 150°C, puis refroidi, dilué par de l'acétate d'éthyle, filtré sur célite, séché sur sulfate de magnésium et concentré sous pression réduite. Le résidu est chromatographié sur une cartouche de silice en éluant par un mélange de dichlorométhane et d'acétate d'éthyle (90/10). Les fractions contenant le produit attendu sont réunies et concentrées à sec sous pression réduite pour donner 110 mg de phényl[6-(1-triisopropylsilyl-pyrrol-3-yl)imidazo[1,2-*a*]pyridin-2-yl]méthanone sous la forme d'une huile verte.

Spectre RMN ¹H (DMSO-d6, δ en ppm) : 1,09 (d, J = 7,5 Hz, 18H), 1,56 (m, 3H), 6,67 (dd, J = 1,5 et 2,0 Hz, 1H), 6,94 (t, J = 2,0 Hz, 1H), 7,40 (s large, 1H), 7,58 (t, J = 7,5 Hz, 2H), 7,67 (m, 2H), 7,78 (dd, J = 1,5 et 9,5 Hz, 1H), 8,33 (d large, J = 7,5 Hz, 2H), 8,51 (s, 1H), 8,82 (s large, 1H).

Spectre de masse (LC-MS-DAD-ELSD) : m/z 444 [M+H]⁺.

### 3.2 : Phényl[6-(1H-pyrrol-3-yl)imidazo[1,2-a]pyridin-2-yl]méthanone

Une solution de 110 mg de phényl[6-(1-triisopropylsilyl-pyrrol-3-yl)imidazo[1,2-*a*]pyridin-2-yl]méthanone dans 1,5 mL de tétrahydrofurane est traitée par 248 µL d'une solution 1M de fluorure de tétrabutylammonium dans le tétrahydrofurane, agitée pendant 2 heures à 25°C puis concentrées sous pression réduite. Le résidu est chromatographié sur une cartouche de silice en éluant par un mélange de dichlorométhane et d'acétate d'éthyle (75/25). Les fractions contenant le produit attendu sont réunies et concentrées à sec sous pression réduite pour donner 31 mg de phényl[6-(1*H*-pyrrol-3-yl)imidazo[1,2-*a*]pyridin-2-yl]méthanone sous la forme d'un solide marron.

### Exemple 4 : Phényl[6-(1H-pyrazol-4-yl)imidazo[1,2-a]pyridin-2-yl]méthanone

Dans un tube à microondes on charge 250 mg (6-iodolimidazo[1,2-*a*]pyridin-2-yl)(phényl)méthanone, 225 mg d'acide 1*H*-4-pyrrazole-boronique, 33 mg de tétrakis-(triphénylphosphine)palladium, 2,5 mL de solution 2M de carbonate de sodium, 5 mL de toluène et 5 mL d'acétonitrile. Le mélange réactionnel est chauffé 20 minutes dans l'appareil à microondes réglé sur 150 °C, puis refroidi, dilué par de l'acétate d'éthyle, filtré sur célite, séché sur sulfate de magnésium et concentré sous pression réduite. Le résidu est chromatographié sur une cartouche de silice en éluant par de l'acétate d'éthyle. Les fractions contenant le produit attendu sont réunies et concentrées à sec sous pression réduite pour donner 15 mg de phényl[6-(1*H*-pyrazol-4-yl)imidazo[1,2-*a*]pyridin-2-yl]méthanone sous la forme d'un solide beige.

### Exemple 5 : (6-(1H-imidazol-4-yl)imidazo[1,2-a]pyridin-2-yl](phényl)méthanone

### 5.1 : Phényl[6-(1-trityl-1H-imidazol-4-yl) imidazo[1,2-a]pyridin-2-yl]méthanone

Dans un tube à microondes on charge 200 mg de phényl[6-(4,4,5,5-tétraméthyl-1,3,2-dioxaborolan-2-yl)imidazo[1,2-*a*]pyridin-2-yl]méthanone, 276 mg de 4-iodo-1-tritylimidazole, 26 mg de tétrakis(triphénylphosphine)palladium, 2 mL de solution 2M de carbonate de sodium, 4 mL de toluène et 4 mL d'acétonitrile. Le mélange réactionnel est chauffé 20 minutes dans l'appareil à microondes réglé sur 150°C, puis refroidi, filtré sur célite, dilué par de l'acétate d'éthyle, séché sur sulfate de magnésium et concentré sous pression réduite. Le résidu est chromatographié sur une cartouche de silice en éluant par un mélange de dichlorométhane et d'acétate d'éthyle (75/25). Les fractions contenant le produit attendu sont réunies et concentrées à sec sous pression réduite. Le solide est trituré dans un mélange de méthanol et de pentane puis séché pour donner 135 mg de phényl[6-(1-trityl-1*H*-imidazol-4-yl) imidazo[1,2-*a*]pyridin-2-yl]méthanone sous la forme d'un solide jaune pâle.

Spectre RMN ¹H (DMSO-d6, δ en ppm) : 7,19 (d, J = 7,5 Hz, 6H), de 7,37 à 7,48 (m, 9H), de 7,51 à 7,60 (m, 4H), 7,65 (m, 2H), 7,79 (dd, J = 1,5 et 9,5 Hz, 1H), 8,32 (d, J = 7,5 Hz, 2H), 8,53 (s, 1H), 9,02 (s large, 1H).
Spectre de masse (LC-MS-DAD-ELSD) : m/z 531 [M+H]⁺.

### 5.2 : [6-(1H-imidazol-4-yl)imidazo[1,2-a]pyridin-2-yl](phényl)méthanone

Une suspension de 133 mg de phényl[6-(1-trityl-1H-imidazol-4-yl) imidazo[1,2-*a*]pyridin-2-yl]méthanone dans un mélange de 5 mL d'acide chlorhydrique 2N et 1 mL d'acide acétique est chauffée au reflux pendant 2,5 heures. Le solide est filtré et lavé par de l'eau chaude. Le filtrat est neutralisé par du carbonate de potassium et extrait par du dichlorométhane. La phase organique est séchée sur sulfate de magnésium et concentrée sous pression réduite. Le solide est séché sous pression réduite pour donner 58 mg de [6-(1*H*-imidazol-4-yl)imidazo[1,2-*a*]pyridin-2-yl](phényl)méthanone sous la forme d'un solide beige.

### Exemple 6 : (6-Furan-2-ylimidazo[1,2-a]pyridin-2-yl)(phényl)méthanone

Dans un tube à microondes on charge 250 mg (6-iodolimidazo[1,2-*a*]pyridin-2-yl)(phényl)méthanone, 843 mg de tributyl-furan-2-yl-stannane, 151 mg de tétrakis-(triphénylphosphine)-palladium et 4 mL de *N,N*-diméthylformamide. Le mélange réactionnel est chauffé 10 minutes dans l'appareil à microondes réglé sur 150°C, puis refroidi et concentré sous pression réduite. Le résidu est chromatographié sur une cartouche de silice en éluant par un mélange de dichlorométhane et d'acétate d'éthyle (90/10). Les fractions contenant le produit attendu sont réunies et concentrées à sec sous pression réduite. Le résidu est cristallisé dans un mélange de méthanol et d'éther diisopropylique pour donner 124 mg de (6-Furan-2-ylimidazo[1,2-a]pyridin-2-yl)(phényl)méthanone sous la forme d'un solide beige.

Les intermédiaires décrits ci-dessous sont utiles à la préparation des composés de la présente invention.

### Intermédiaire 1 : (6-Iodoimidazo[1,2-a]pyridin-2-yl)(phényl)méthanone

A une solution de 4,5 g de 2-amino-5-iodopyridine dans 35 mL de tétrahydrofurane on ajoute une solution de 5,5 g de 3-bromo-1-phényl-propane-1,2-dione dans 35 mL de tétrahydrofurane. Le mélange réactionnel est agité 16 heures à 20°C puis concentré à sec sous pression réduite. Le résidu est repris dans 100mL d'éthanol et chauffé au reflux pendant 2,5 heures. Le mélange réactionnel est concentré à sec et repris dans une solution saturée de bicarbonate de sodium et du dichlorométhane. La phase organique est séchée sur sulfate de magnésium et évaporée à sec. Le solide est trituré dans de l'éther éthylique, filtré et séché pour donner 4,14 g de (6-iodoimidazo[1,2-*a*]pyridin-2-yl)(phényl)méthanone sous la forme d'un solide jaune orangé.

Spectre RMN 1H (DMSO-d6, δ en ppm) : 7,58 (t, J=7,6 Hz, 4H), 7,67 (d, J=7,3 Hz, 1H), 8,30 (d, J=7,8 Hz, 1H), 8,54 (s, 1H), 9,01 (s, 1H).

Spectre de masse (LC-MS-DAD-ELSD) : m/z 349 : [M+H]⁺.

L'intermédiaire 1 (6-iodoimidazo[1,2-*a*]pyridin-2-yl)(phényl)méthanone) est déjà décrit dans le document WO 2008/003854 (composé 22 du Tableau). A ce titre, il est exclu de la portée de la présente invention.

### Intermédiaire 2 : Phényl[6-(4,4,5,5-tétraméthyl-1,3,2-dioxaborolan-2-yl)imidazo[1,2-a]pyridin-2-yl]méthanone

A une solution de 7 g de 2-amino-5-(4,4,5,5-tétraméthyl-1,3,2-dioxaborolan-2-yl)pyridine dans 50 mL de tétrahydrofurane on ajoute une solution de 6 g de 3-bromo-1-phényl-propane-1,2-dione dans 50 mL de tétrahydrofurane. Le mélange réactionnel est agité 16 heures à température ambiante puis concentré à sec sous pression réduite. Le résidu est repris dans 100 mL d'éthanol et chauffé au reflux pendant 2,5 heures. Après concentration à sec sous pression réduite, le solide est repris dans une solution saturée de bicarbonate de sodium et du dichlorométhane. La phase organique est séchée sur sulfate de magnésium et évaporée à sec Le résidu est redissout dans 30 mL d'éthanol et 12 mL d'acide chlorhydrique 2N. Le mélange réactionnel est agité à 25°C puis évaporé à sec et repris dans du dichlorométhane et 24 mL de soude 1N. La phase organique est séchée sur sulfate de magnésium et évaporée à sec. Le résidu est trituré dans un mélange de dichlorométhane et d'éther éthylique. L'insoluble est filtré et le filtrat concentré trituré dans un mélange de dichlorométhane, méthanol et d'éther éthylique. Le solide est filtré et séché pour donner 1,2 g de phényl[6-(4,4,5,5-tétraméthyl-1,3,2-dioxaborolan-2-yl)imidazo[1,2-*a*]pyridin-2-yl]méthanone sous la forme d'un solide beige orangé.

Spectre RMN 1H (DMSO-d6, δ en ppm) : 1,33 (s, 12H), 7,44 (dd, J = 1,5 et 9,5 Hz, 1H), 7,58 (t, J = 7,5 Hz, 2H), 7,69 (m, 3H), 8,30 (d, J = 7,5 Hz, 2H), 8,70 (s, 1H), 8,95 (s large, 1H).

Spectre de masse (IE) : m/z 348 : [M]⁺.

Les tableaux qui suivent illustrent les structures chimiques (tableau 1) et les caractéristiques spectroscopiques (tableau 2) de quelques exemples de composés selon l'invention.

Dans la colonne « sel » du tableau 1, « - » représente un composé sous forme de base libre, alors que « HCl » représente un composé sous forme de chlorhydrate et le rapport entre parenthèses est le rapport (acide:base),

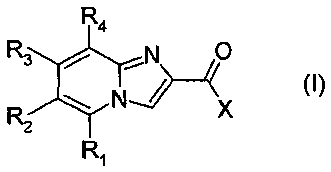

**Tableau 1**

| **Ex** | **R₁** | **R₂** | **R₃** | **R₄** | **X** | **Sel** |
|---|---|---|---|---|---|---|
| **1** | H | 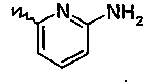 | H | H | 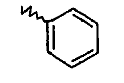 | - |
| **2** | H | 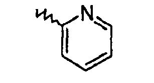 | H | H | 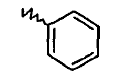 | HCl (2:1) |
| **3** | H | 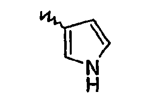 | H | H | 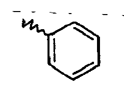 | - |
| **4** | H | 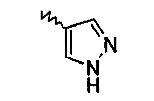 | H | H | 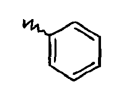 | - |
| **5** | H | 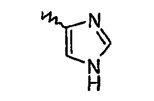 | H | H | 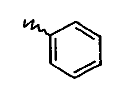 | - |
| **6** | H | 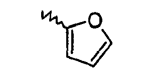 | H | H | 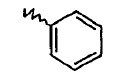 | - |
| 7 | H | 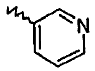 | H | H | 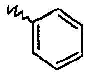 | - |

**Tableau 2**

| **Composé** | **Caractérisations** |
|---|---|
| 1 | Spectre RMN ¹H (DMSO-d6, δ en ppm) : 6.08 (s large, 2 H), 6.49 (d, J=7.8 Hz, 1 H), 7.10 (d, J=7.8 Hz, 1H), 7.52 (t, J=7.8 Hz, 1 H), 7.59 (t, J=7.6 Hz, 2 H), 7.68 (t, J=7.6 Hz, 1 H), 7.76 (d, J=9.8 Hz, 1 H), 7.99 (dd, J=9.8, 2.0 Hz, 1H), 8.33 (d large, J=7.8 Hz, 2 H), 8.72 (s, 1 H), 9.20 (s large, 1 H). |
| | Spectre de masse (LC-MS-DAD-ELSD) : m/z 315 [M+H]⁺. |
| 2 | Spectre RMN ¹H (DMSO-d6, δ en ppm) : 7,49 (dd large, J = 5,0 et 80, Hz 1H), 7,64 (t, J = 7,5 Hz, 2H), 7,76 (t, J = 7,5 Hz, 1H), 7,94 (d, J = 9,5 Hz, 1H), 8,03 (dt, J = 1,5 et 8,0 Hz, 1H), 8,09 (d, J = 8,0 Hz, 1H), 8,22 (d, J = 7,5 Hz, 2H), 8,40 (d large, J = 9,5 Hz, 1H), 8,75 (d, J = 5,0 Hz, 1H), 8,91 (s, 1H), 9,54 (s large, 1H). |
| | Spectre de masse (LC-MS-DAD-ELSD) : m/z 300 [M+H]⁺. |
| 3 | Spectre RMN ¹H (DMSO-d6, δ en ppm) : 6,47 (dd, J = 1,5 et 2,0 Hz, 1H), 6,87 (dd, J = 1,0 et 2,0 Hz, 1H), 7,32 (dd, J = 1,0 et 1,5 Hz, 1H), 7,59 (t, J = 7,5 Hz, 2H), de 7,62 à 7,73 (m, 3H), 8,32 (d, J = 7,5 Hz, 2H), 8,53 (s, 1H), 8,77 (s large, 1H), 11,05 (m étalé, 1H). |
| | Spectre de masse (LC-MS-DAD-ELSD) : m/z 286 [M-H]⁺, m/z 288 [M+H]⁺. |
| 4 | Spectre RMN ¹H (DMSO-d6, δ en ppm) : 7,59 (t, J = 7,5 Hz, 2H), 7,69 (t, J = 7,5 Hz, 1H), 7,72 (m, 2H), 8,13 (m étalé, 2H), 8,32 (d, J = 7,5 Hz, 2H), 8,53 (s, 1H), 8,91 (s large, 1H), 13,05 (m étalé, 1H). |
| | Spectre de masse (LC-MS-DAD-ELSD) : m/z 287 [M-H]⁺, m/z 289 [M+H]⁺, |
| 5 | Spectre RMN ¹H (DMSO-d6, δ en ppm) : 7,59 (t, J = 7,5 Hz, 2H), 7,68 (t, J = 7,5 Hz, 1H), 7,72 (m, 2H), 7,80 (m, 2H), 8,33 (d, J = 7,5 Hz, 2H), 8,68 (s, 1H), 9,00 (s large, 1H), 12,3 (m étalé, 1H). |
| | Spectre de masse (LC-MS-DAD-ELSD) : m/z 287 [M-H]⁺, m/z 289 [M+H]⁺. |
| 6 | Spectre RMN ¹H (DMSO-d6, δ en ppm) : 6.66 (dd, J=3.4, 2.0 Hz, 1H) 7.07 (d large, J=3.4 Hz, 1H) 7.58 (t, J=7.3 Hz, 2H) 7.68 (tt, J=7.3, 1.4 Hz, 1H) 7.73 - 7.81 (m, 2H) 7.83 (d large, J=2.0 Hz, 1H) 8.33 (m, 2H) 8.69 (s, 1H) 8.98 (s large, 1H). |
| | Spectre de masse (LC-MS-DAD-ELSD): m/z 289-[M+H]⁺. |
| 7 | Spectre RMN ¹H (DMSO-d6, δ en ppm) : 7,56 (dd, J = 5,0 et 8,0 Hz, 1H), 7,60 (t, J = 7,5 Hz, 2H), 7,70 (tt, J =1,5 et 7,5 Hz, 1H), 7,81 (dd, J = 1,5 et 9,5 Hz, 1H), 7,86 (d, J = 9,5 Hz, 1H), 8,16 (td, J = 1,0 et 8,0 Hz, 1H), 8,34 (d large, J = 7,5 Hz, 2H), 8,53 (m, 2H), 8,98 (d, J = 2,0 Hz, 1H), 9,09 (s large, 1H). |
| | Spectre de masse (LC-MS-DAD-ELSD) : m/z 322 [M+Na]⁺. |

Les composés selon l'invention ont fait l'objet d'essais pharmacologiques permettant de déterminer leur effet modulateur sur NOT.

### Evaluation de l'activité in vitro sur cellules N2A

L'activité des composés selon l'invention a été évaluée sur une lignée cellulaire (N2A) exprimant de manière endogène le récepteur de souris Nurrl et transfectée de manière stable avec l'élément de réponse liant NOT (NBRE) couplé au gène rapporteur luciférase. Les EC₅₀ sont comprises entre 0,01 et 1000 nM. Les essais ont été réalisés selon le mode opératoire décrit ci dessous.

La lignée cellulaire Neuro-2A provient de source commerciale standard (ATCC). Le clone Neuro-2A a été obtenu à partir d'une tumeur spontanée provenant d'une souche de souris A albino par R.J Klebe et col. Cette lignée Neuro-2A est ensuite stablement transfectée avec 8NBRE-luciférase. Les cellules N2A-8NBRE sont cultivées jusqu'à confluence dans des flacons de culture de 75 cm² contenant du DMEM supplémenté par 10% de sérum foetal de veau, 4,5 g/L de glucose et 0,4 mg/ml de Généticine. Après une semaine de culture les cellules sont récupérées par de la trypsine 0,25% pendant 30 secondes puis remises en suspension dans du DMEM sans rouge de phénol contenant 4,5g/L de glucose, 10% de sérum délipidé Hyclone et déposées dans des plaques blanches 96 puits à fond transparent. Les cellules sont déposées à raison de 60.000 par puits dans 75 µL pendant 24 heures avant l'addition des produits. Les produits sont appliqués dans 25µL et incubés 24 heures supplémentaires. Le jour de la mesure, on ajoute à chaque puits un volume équivalent (100µL) de Steadylite, puis on attend 30 minutes pour obtenir une lyse complète des cellules et la production maximale du signal. Les plaques sont ensuite mesurées dans un compteur de luminescence pour microplaques après avoir été scellées par un film adhésif. Les produits sont préparés sous forme de solution stock à 10⁻² M, puis dilués dans 100% de DMSO. Chaque concentration de produit est préalablement diluée dans du milieu de culture avant incubation avec les cellules contenant ainsi 0,625% final de DMSO.

Par exemple, les composés n° 1, 4 et 7 ont montré une CE₅₀ de 0,7, 0,5 et 0,5 nM respectivement.

Il apparaît donc que les composés selon l'invention ont un effet modulateur de NOT.

Les composés selon l'invention peuvent donc être utilisés pour la préparation de médicaments pour leur application en thérapeutique dans le traitement ou la prévention de maladies impliquant les récepteurs NOT.

Ainsi, selon un autre de ses aspects, l'invention a pour objet des médicaments qui comprennent un composé de formule (I), ou un sel d'addition de ce dernier à un acide pharmaceutiquement acceptable.

Ces médicaments trouvent leur emploi en thérapeutique, notamment dans le traitement et la prévention des maladies neurodégénératives telles que par exemple la maladie de Parkinson, d'Alzheimer, les tauopathies (ex. la paralysie progressive supranucléaire, la démence fronto temporale, la dégénérescence corticobasale, la maladie de Pick); les traumatismes cérébraux comme l'ischémie et les traumatismes crâniens et l'épilepsie ; les maladies psychiatriques comme la schizophrénie, la dépression, la dépendance à une substance, les troubles du déficit de l'attention et de l'hyperactivité ; les maladies inflammatoires du système nerveux central comme la sclérose en plaque, encéphalite, myélite et encéphalomyélite et autres maladies inflammatoires comme les pathologies vasculaires, l'athérosclérose, les inflammations des articulations, l'arthrose, l'arthrite rhumatoïde ; l'ostéoarthrite, la maladie de Crohn, colite ulcéreuse; les maladies inflammatoires allergiques telle que l'asthme, les maladies auto-immunes comme le diabète de type 1, lupus, sclérodermies, Syndrome de Guillain-Barré, maladie d'Addison et autres maladies immuno-médiées; l'ostéoporose; les cancers.

Ces composés pourraient être aussi utilisés comme traitement associé à des greffes et/ou transplantations de cellules souches.

Ainsi, un objet de la présente invention vise un composé de formule (I) tel que défini précédemment, pour le traitement des maladies et désordres précités.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscarmellose sodique | 6,0 mg |
| Amidon de maïs | 15,0 mg |
| Hydroxypropyl-méthylcellulose | 2,25 mg |
| Stéarate de magnésium | 3,0 mg |

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

## Revendications

1. Composés de formule (I) : 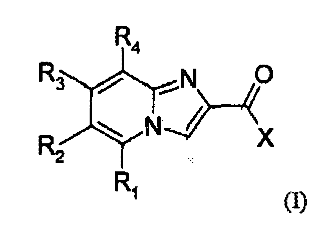 dans laquelle :
X représente un groupe phényle éventuellement substitué par un ou plusieurs atomes ou groupes choisis indépendamment les uns des autres parmi les atomes ou groupes suivants : halogène, (C₁-C₆)alkyle, (C₁-C₆)alcoxy, hydroxy, amino, NRaRb ; les groupes (C₁-C₆)alkyle et (C₁-C₆)alcoxy étant éventuellement substitués par un ou plusieurs atomes d'halogène ;
R₂ représente un groupe hétérocyclique éventuellement substitué par un ou plusieurs groupes choisis indépendamment les uns des autres parmi les atomes ou groupes suivantes : hydroxy, halogène, (C₁-C₆)alcoxy, (C₁-C₆)alkyle éventuellement substitué par un ou plusieurs hydroxy, NRcRd, -CO-R₅, -CO-NR₆R₇, -CO-O-R₈, -NR₉-CO-R₁₀, cyano, un groupe oxido ;
R₁ représente un atome d'hydrogène, un halogène, un groupe (C₁-C₆)alcoxy, un groupe (C₁-C₆)alkyle, hydroxy, amino; les groupes (C₁-C₆)alkyle et (C₁-C₆)alcoxy pouvant éventuellement être substitués par un ou plusieurs atomes ou groupes halogène, hydroxy, amino, (C₁-C₆)alcoxy;
R₃ représente un atome d'hydrogène, un groupe (C₁-C₆)alkyle, un atome d'halogène, ou un groupe hydroxy;
R₄ représente un atome d'hydrogène ou un atome d'halogène ;
R₅ représente un atome d'hydrogène ou un groupe (C₁-C₆)alkyle ;
R₆ et R₇, identiques ou différents, représentent un atome d'hydrogène ou un groupe (C₁-C₆)alkyle ou forment avec l'atome d'azote qui les porte un cycle de 4 à 7 chaînons incluant éventuellement un autre hétéroatome choisi parmi N, O ou S ;
R₈ représente un groupe (C₁-C₆)alkyle;
R₉ et R₁₀, identiques ou différents, représentent un atome d'hydrogène ou un groupe (C₁-C₆)alkyle ; Ra représente un (C₁-C₆)alkyle ; Rb, Rc et Rd représentent un hydrogène ou un (C₁-C₆)alkyle ;
un groupe hétérocyclique représente un groupe mono ou bicyclique saturé ou insaturé ou
partiellement insaturé comportant de 5 à 10 atomes dont de 1 à 4 hétéroatomes choisis parmi N, O et S ;
à l'état de base ou de sel d'addition à un acide.

2. Composés de formule (I) selon la revendication 1, **caractérisé en ce que**
X représente un groupe phényle ;
R₁, R₃ et R₄ sont des atomes d'hydrogène ;
R₂ représente un groupe hétérocyclique monocyclique insaturé comportant 5 ou 6 atomes dont de 1 à 2 hétéroatomes choisis parmi N ou O, ledit groupe hétérocyclique étant éventuellement susbtitué par un groupe -NR_{c}R_{d}, R_{c} et R_{d} représentant un hydrogène ou un (C₁-C₆)alkyle.

3. Composé de formule (I) selon les revendications 1 ou 2, **caractérisé en ce que**
X représente un groupe phényle ;
R₁, R₃ et R₄ sont des atomes d'hydrogène ;
R₂ représente un groupe pyridine, pyrrole, pyrazole, imidazole ou furane, éventuellement substitué par un groupe NH₂;
à l'état de base ou de sel d'addition à un acide.

4. Composé de formule (I) selon les revendications 1 ou 2 choisi parmi :
[6-(6-Aminopyridin-2-yl)imidazo[1,2-*a*]pyridin-2-yl](phényl)méthanone,
Phényl(6-pyridin-2-ylimidazo[1,2-*a*]pyridin-2-yl)méthanone et son dichlorhydrate,
Phényl[6-(1H-pyrrol-3-yl)imidazo[1,2-*a*]pyridin-2-yl]méthanone,
Phényl[6-(1H-pyrazol-4-yl)imidazo[1,2-*a*]pyridin-2-yl]méthanone,
[6-(1H-imidazol-4-yl)imidazo[1,2-*a*]pyridin-2-yl](phényl)méthanone,
[(6-Furan-2-yl)imidazo[1,2-*a*]pyridin-2-yl](phényl)méthanone,
Phényl[(6-pyridin-3-yl)imidazo[1,2-*a*]pyridin-2-yl]méthanone,
ou les sels d'addition de ces composés à un acide pharmaceutiquement acceptable.

5. Médicament, **caractérisé en ce qu'**il comprend un composé de formule (I) selon l'une quelconque des revendications 1 à 4, ou un sel d'addition de ce composé à un acide pharmaceutiquement acceptable.

6. Composition pharmaceutique, **caractérisée en ce qu'**elle comprend un composé de formule (I) selon l'une quelconque des revendications 1 à 4, ou un sel pharmaceutiquement acceptable, de ce composé, ainsi qu'au moins un excipient pharmaceutiquement acceptable.

7. Utilisation d'un composé de formule (I) selon l'une quelconque des revendications 1 à 4 pour la préparation d'un médicament destiné au traitement et à la prévention des maladies neurodégénératives.

8. Utilisation d'un composé de formule (I) selon l'une quelconque des revendications 1 à 4 pour la préparation d'un médicament destiné au traitement et à la prévention des traumatismes cérébraux et de l'épilepsie.

9. Utilisation d'un composé de formule (I) selon l'une quelconque des revendications 1 à 4 pour la préparation d'un médicament destiné au traitement et à la prévention des maladies psychiatriques.

10. Utilisation d'un composé de formule (I) selon l'une quelconque des revendications 1 à 4 pour la préparation d'un médicament destiné au traitement et à la prévention des maladies inflammatoires.

11. Utilisation d'un composé de formule (I) selon l'une quelconque des revendications 1 à 4 pour la préparation d'un médicament destiné au traitement et à la prévention de l'ostéoporose et les cancers.

12. Utilisation d'un composé de formule (I) selon l'une quelconque des revendications 1 à 4 pour la préparation d'un médicament destiné au traitement et à la prévention de la maladie de Parkinson, d'Alzheimer, des tauopathies, de la sclérose en plaque.

13. Utilisation d'un composé de formule (I) selon l'une quelconque des revendications 1 à 4 pour la préparation d'un médicament destiné au traitement et à la prévention de la schizophrénie, la dépression, la dépendance à une substance les troubles du déficit de l'attention et de l'hyperactivité;

14. Composé Phényl[6-(4,4,5,5-tétraméthyl-1,3,2-dioxaborolan-2-yl)imidazo[1,2-*a*]pyridin-2-yl]méthanone

15. Utilisation des composés selon la revendication 14 pour la synthèse de produits de formule générale (I) telle que définie dans la revendication 1 à 4.

## Claims

1. Compounds of formula (I): 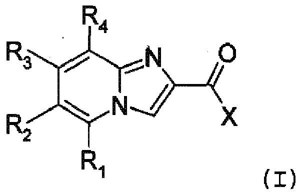 in which:
X is a phenyl group optionally substituted with one or more atoms or groups chosen, independently of one another, from the following atoms or groups: halogen, (C₁-C₆) alkyl, (C₁-C₆) alkoxy, hydroxyl, amino, NRaRb; the (C₁-C₆)alkyl and (C₁-C₆)alkoxy groups being optionally substituted with one or more halogen atoms;
R₂ is a heterocyclic group optionally substituted with one or more groups chosen, independently of one another, from the following atoms or groups: hydroxyl, halogen (C₁-C₆)alkoxy, (C₁-C₆)alkyl optionally substituted with one or more hydroxyl NRcRd, -CO-R₅, -CO-NR₆R₇, -CO-O-R₈, -NR₉-CO-R₁₀, cyano, and an oxido group;
R₁ is a hydrogen atom, a halogen, a (C₁-C₆)alkoxy group, a (C₁-C₆)alkyl group, hydroxyl or amino; it being possible for the (C₁-C₆) alkyl and (C₁-C₆)alkoxy groups to be optionally substituted with one or more of the following atoms or groups: halogen, hydroxyl, amino, (C₁-C₆)alkoxy;
R₃ is a hydrogen atom, a halogen atom, a (C₁-C₆)alkyl group or a hydroxyl group;
R₄ is a hydrogen atom or a halogen atom;
R₅ is a hydrogen atom or a (C₁-C₆)alkyl group;
R₆ and R₇, which may be identical or different, are a hydrogen atom or a (C₁-C₆)alkyl group, or form, with the nitrogen atom which bears them, a 4- to 7-membered ring optionally including another heteroatom chosen from N, O or S;
R₈ is a (C₁-C₆)alkyl group;
R₉ and R₁₀, which may be identical or different, are a hydrogen atom or a (C₁-C₆)alkyl group; Ra is a (C₁-C₆)alkyl; Rb, Rc and Rd are a hydrogen atom or a (C₁-C₆)alkyl ; a heterocyclic group is a saturated or unsaturated or partially unsaturated, monocyclic or bicyclic group containing from 5 to 10 atoms, including from 1 to 4 heteroatoms chosen from N, O and S; in the form of a base or of an addition salt with an acid.

2. Compounds of formula (I) according to Claim 1, **characterized in that**:
X is a phenyl group;
R₁, R₃ and R₄ are hydrogen atoms;
R₂ is an unsaturated monocyclic heterocyclic group containing 5 or 6 atoms, including from 1 to 2 heteroatoms chosen from N or O, said heterocyclic group being optionally substituted with an -NR_{c}R_{d} group, R_{c} and R_{d} being a hydrogen or a (C₁-C₆)alkyl.

3. Compound of formula (I) according to Claim 1 or 2, **characterized in that**:
X is a phenyl group;
R₁, R₃ and R₄ are hydrogen atoms;
R₂ is a pyridine, pyrrole, pyrazole, imidazole or furan group, optionally substituted with an NH₂ group;
in the form of a base or of an addition salt with an acid.

4. Compound of formula (I) according to Claim 1 or 2, chosen from:
[6-(6-aminopyridin-2-yl)imidazo[1,2-*a*]pyridin-2-yl](phenyl)methanone,
phenyl(6-pyridin-2-ylimidazo[1,2-*a*]pyridin-2-yl)methanone and the dihydrochloride thereof,
phenyl[6-(1H-pyrrol-3-yl)imidazo[1,2-*a*]pyridine-2-yl]methanone,
phenyl[6-(1H-pyrazol-4-yl)imidazo[1,2-*a*]pyridin-2-yl]methanone,
[6-(1H-imidazol-4-yl)imidazo[1,2-*a*]pyridin-2-yl](phenyl)methanone,
[(6-furan-2-yl)imidazo[1,2-*a*]pyridin-2-yl](phenyl)methanone,
phenyl[(6-pyridin-3-yl)imidazo[l,2-*a*]pyridin-2-yl]methanone,
or the addition salts of these compounds with a pharmaceutically acceptable acid.

5. Medicament, **characterized in that** it comprises a compound of formula (I) according to any one of Claims 1 to 4, or an addition salt of this compound with a pharmaceutically acceptable acid.

6. Pharmaceutical composition **characterized in that** it comprises a compound of formula (I) according to any one of Claims 1 to 4, or a pharmaceutically acceptable salt of this compound, and also at least one pharmaceutically acceptable excipient.

7. Use of a compound of formula (I) according to any one of Claims 1 to 4, for the preparation of a medicament for use in the treatment and prevention of neurodegenerative diseases.

8. Use of a compound of formula (I) according to any one of Claims 1 to 4, for the preparation of a medicament for use in the treatment and prevention of cerebral traumas and of epilepsy.

9. Use of a compound of formula (I) according to any one of Claims 1 to 4, for the preparation of a medicament for use in the treatment and prevention of psychiatric diseases.

10. Use of a compound of formula (I) according to any one of Claims 1 to 4, for the preparation of a medicament for use in the treatment and prevention of inflammatory diseases.

11. Use of a compound of formula (I) according to any one of Claims 1 to 4, for the preparation of a medicament for use in the treatment and prevention of osteoporosis and cancers.

12. Use of a compound of formula (I) according to any one of Claims 1 to 4, for the preparation of a medicament for use in the treatment and prevention of Parkinsons disease, Alzheimer's disease, tauopathies and multiple sclerosis.

13. Use of a compound of formula (I) according to any one of Claims 1 to 4, for the preparation of a medicament for use in the treatment and prevention of schizophrenia, depression, substance dependence and attention deficit hyperactivity disorders.

14. Compound phenyl[6-(4,4,5,5-tetramethyl-1,3,2-dioxaborolan-2-yl)imidazo[1,2-*a*]pyridin-2-yl]methanone.

15. Use of the compound according to Claim 14, for the synthesis of products of general formula (I) as defined in Claims 1 to 4.

## Patentansprüche

1. Verbindungen der Formel (I): 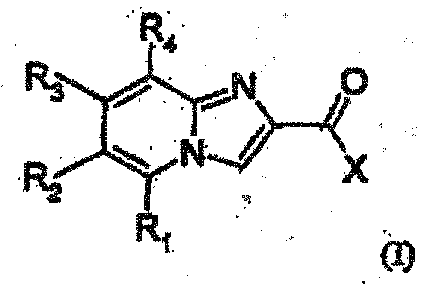 worin:
X für eine Phenylgruppe steht, die gegebenenfalls durch ein oder mehrere Atome bzw. eine oder
mehrere Gruppen substituiert ist, die unabhängig voneinander unter den folgenden Atomen bzw.
Gruppen ausgewählt sind: Halogen, (C₁-C₆)-Alkyl, (C₁-C₆)-Alkoxy, Hydroxy, Amino und NRaRb; wobei die (C₁-C₆) -Alkyl- und (C₁-C₆) -Alkoxygruppen gegebenenfalls durch ein oder mehrere Halogenatome substituiert sind;
R₂ für eine heterocyclische Gruppe steht, die gegebenenfalls durch eine oder mehrere Gruppen substituiert ist, die unabhängig voneinander unter den folgenden Atomen bzw. Gruppen ausgewählt sind: Hydroxy, Halogen, (C₁-C₆) -Alkoxy, (C₁-C₆) -Alkyl, gegebenenfalls substituiert durch ein oder mehrere Hydroxy, NRcRd, -CO-R₅, -CO-NR₆R₇, -CO-O-R₈, -NR₉-CO-R₁₀, Cyano, eine Oxidogruppe;
R₁ für ein Wasserstoffatom, ein Halogen, eine (C₁-C₆)-Alkoxygruppe, eine (C₁-C₆) -Alkylgruppe, ein Hydroxy oder ein Amino steht; wobei die (C₁-C₆)-Alkyl- und (C₁-C₆)-Alkoxygruppen gegebenenfalls durch ein bzw. eine oder mehrere Halogenatome, Hydroxygruppen, Aminogruppen und/oder (C₁-C₆)-Alkoxygruppen substituiert sind;
R₃ für ein Wasserstoffatom, eine (C₁-C₆)-Alkylgruppe, ein Halogenatom oder eine Hydroxygruppe steht;
R₄ für ein Wasserstoffatom oder ein Halogenatom steht;
R₅ für ein Wasserstoffatom oder eine (C₁-C₆)-Alkylgruppe steht;
R₆ und R₇ gleich oder verschieden sind und für ein Wasserstoffatom oder eine (C₁-C₆) -Alkylgruppe stehen oder mit dem Stickstoffatom, an das sie gebunden sind, einen 4- bis 7-gliedrigen Ring, der gegebenenfalls ein weiteres unter N, O oder S ausgewähltes Heteroatom enthält, bildern;
R₈ für eine (C₁-C₆)-Alkylgruppe steht;
R₉ und R₁₀ gleich oder verschieden sind und für ein Wasserstoffatom oder eine (C₁-C₆)-Alkylgruppe stehen;
Ra für (C₁-C₆)-Alkyl steht;
Rb, Rc und Rd für Wasserstoff oder (C₁-C₆)-Alkyl stehen;
eine heterocyclische Gruppe für eine gesättigte oder ungesättigte oer teilweise ungesättigte mono- oder bicyclische Gruppe mit 5 bis 10 Atomen einschließlich 1 bis 4 Heteroatomen, die unter N, O und S ausgewählt sind, steht;
in Basen- oder Säueradditionssalzform.

2. Verbindungen der Formel (I) nach Anspruch 1,
**dadurch gekennzeichnet, dass**
X für eine Phenylgruppe steht;
R₁, R₃ und R₄ für Wasserstoffatome stehen;
R₂ für eine ungesättigte monocyclische heterocyclische Gruppe mit 5 oder 6 Atomen einschließlich 1 bis 2 Heteroatomen, die unter N oder O ausgewählt sind, steht, wobei die heterocyclische Gruppe gegebenenfalls durch eine Gruppe -NR_{c}R_{d} substituiert ist, wobei R_{c} und R_{d} für Wasserstoff oder (C₁-C₆)-Alkyl stehen.

3. Verbindung der Formel (I) nach Anspruch 1 oder 2,
**dadurch gekennzeichnet, dass**
X für eine Phenylgruppe steht;
R₁, R₃ und R₄ für Wasserstoffatome stehen;
R₂ für eine Pyridin-, Pyrrol-, Pyrazol-, Imidazol- oder Furangruppe steht, die gegebenenfalls durch eine Gruppe -NH₂ substituiert ist;
in Basen- oder Säveradditionssalzform.

4. Verbindung der Formel (I) nach Anspruch 1 oder 2, ausgewählt unter:
[6-(6-Aminopyridin-2-yl)imidazo[1,2-*a*]pyridin-2-yl](phenyl)methanon,
Phenyl(6-pyridin-2-ylimidazo[1,2-*a*]pyridin-2-yl)methanon und dessen Dihydrochlorid,
phenyl[6-(1H-pyrrol-3-yl)imidazo[1,2-*a*]pyridin-2-yl] methanon,
Phenyl[6-(1H-pyrazol-4-yl)imidazo[1,2-*a*]pyridin-2-yl]methanon,
[6-(1H-Imidazol-4-yl)imidazo[1,2-*a*]pyridin-2-yl]-(phenyl)methanon,
[(6-Furan-2-yl)imidazo[1,2-*a*]pyridin-2-yl]-(phenyl)methanon,
Phenyl[(6-pyridin-3-yl)imidazo[1,2-*a*]pyridin-2-yl]methanon,
oder die Additionssalze dieser Verbindungen mit einer pharmazeutisch unbedenklichen Säure.

5. Medikament, **dadurch gekennzeichnet, dass** es eine Verbindung der Formel (I) nach einem der Ansprüche 1 bis 4 oder ein Additionssalz dieser Verbindung mit einer pharmazeutisch unbedenklichen Säure enthält.

6. Pharmazeutische Zusammensetzung, **dadurch gekennzeichnet, dass** sie eine Verbindung der Formel (I) nach einem der Ansprüche 1 bis 4 oder ein pharmazeutisch unbedenkliches Salz dieser Verbindung sowie mindestens einen pharmazeutisch unbedenklichen Hilfsstoff enthält.

7. Verwendung einer Verbindung der Formel (I) nach einem der Ansprüche 1 bis 4 zur Herstellung eines Medikaments zur Behandlung und Prävention von neurodegenerativen Erkrankungen.

8. Verwendung einer Verbindung der Formel (I) nach einem der Ansprüche 1 bis 4 zur Herstellung eines Medikaments zur Behandlung und Prävention von Hirntraumen und Epilepsie.

9. Verwendung einer Verbindung der Formel (I) nach einem der Ansprüche 1 bis 4 zur Herstellung eines Medikaments zur Behandlung und Prävention von psychiatrischen Erkrankungen.

10. Verwendung einer Verbindung der Formel (I) nach einem der Ansprüche 1 bis 4 zur Herstellung eines Medikaments zur Behandlung und Prävention von entzündlichen Erkrankungen.

11. Verwendung einer Verbindung der Formel (I) nach einem der Ansprüche 1 bis 4 zur Herstellung eines Medikaments zur Behandlung und Prävention von Osteoporose und Krebserkrankungen.

12. Verwendung einer Verbindung der Formel (I) nach einem der Ansprüche 1 bis 4 zur Herstellung eines Medikaments zur Behandlung und Prävention von Parkinson-Krankheit, Alzheimer-Krankheit, Tauopathien und multipler Sklerose.

13. Verwendung einer Verbindung der Formel (I) nach einem der Ansprüche 1 bis 4 zur Herstellung eines Medikaments zur Behandlung und Prävention von Schizophrenie, Depression, Substanzabhängigkeit und Aufmerksamkeitsdefizit-Hyperaktivitätssyndrom.

14. Verbindung Phenyl[6-(4,4,5,5-tetramethyl-1,3,2-dioxaborolan-2-yl)imidazo[1,2-*a*]pyridin-2-yl]methanon.

15. Verwendung von Verbindungen nach Anspruch 14 zur Synthese von Produkten der allgemeinen Formel (I) gemäß den Ansprüchen 1 bis 4.
